# EUROPEAN PATENT APPLICATION

(11) **EP 2 169 630 A1**
(43) Date of publication of application: **31.03.2010**
(21) Application number: 08105394.4
(22) Date of filing: 19.09.2008
(51) Int. Cl.: G07C 3/00, A61G 7/00

(54) **Activity log system**

(71) Applicant: Arjo Hospital Equipment AB, 241 21 Eslöv (SE)
(72) Inventor: Jonsson, Jörgen, SE-223 70 Lund (SE)
(74) Representative: Thaker, Shalini

(57) **Abstract**

An activity log system for patient handling equipment, for example a hoist, comprises an accelerometer (3) mounted on the patient handling equipment. The accelerometer together with a microprocessor logs data over time, so that movement of the equipment is measured and recorded. The data collected is transmitted to a main control unit, in contact with other activity log systems, which watches logged data with experience data to enable mapping of equipment usage over time.

## Description

The present invention relates to an activity log system for patient handling equipment, in particular lifting handling equipment transporting patients.

Most of the Carers working in healthcare today are aware of the need of mechanical aids when lifting and transferring patients. Even with this awareness, a lot of Carers have problems with back injuries.

There is a need for the hospitals to minimize these problems, by investigating whether the hospital has the right mechanical aids, enough equipment, equipment that is easy to use and easy to get hold of, the right level of education and training for the Carers and whether these processes are in place at the establishment.

Many care facilities are planning to invest further in programs and equipment, in order to reduce back injuries among their employees. One way of ensuring that the hospitals invest money in the right processes, equipment and training would be to monitor the usage of patient handling equipment throughout a care facility over time, and based on that guide decide on how best to improve their processes, equipment and training.

Patient handling equipment of today offer some degree of data logging internally mainly for the purpose of servicing the equipment, this information is difficult to collect for any other person other than service personnel. Furthermore this information does not disclose the type of activity the equipment has been involved in.

The present invention seeks to make improvements. Accordingly, the present invention provides an activity log system for a patient handling equipment, including an accelerometer together with a microprocessor and a transceiver powered from a power source mounted on the equipment, the accelerometer data relating to movement of the equipment collected and interpreted to identify specific equipment activities.

The present invention solves the problem of obtaining coherent data from a flora of different patient handling equipment, from different manufacturers and different ages without compromising the function or integrity of the supervised equipment. The invention can identify the type of activity undertaken by the patient handling equipment even if the activity is manually driven, as a trolley for example.

The invention answers the need for identifying the kind of activity undertaken with a piece of equipment.

Preferably, specific event data is identified by matching derived sensor data against experience data. The system is not tied to a specific product, since it is an autonomous device. The sensor can be added to any lift equipment without any influence on the medical device approval. The data is made readily accessible to a main control unit.

Preferably, a main control unit, in contact with the autonomous activity log units, matches logged data with extended data, such as number of personnel and patients at a given time, to gain knowledge for decisions.

The system sorts out a unique pattern that matches the event (1) identified to be monitored. This pattern is built on experience values from trials, the number of trials determining the accuracy of the system.
(1) Specific event could be (but not limited to):
   - A complete patient transfer
   - A repositioning of a patient
   - A limb holding event of a patient
   - A short movement without patient involvement
   - A transport of the equipment to a storage place
(2) Measuring data could be (but not limited to):
   - Accelerometer data
   - Gyroscopic data
   - Microphone listening for sound from the actuator motor
   - Hall effect device sensing the magnetic field generated by the actuator motor
(3) Collected data that are written to a log could be (but not limited to):
   - Sort of event identified
   - Time of day and date
   - ID-number of equipment
   - Event length

The system has the advantage over normal "on-board" logging in hoists and lifters, in that it:
Sorts out a specific event by matching derived sensor data against experience data.
Is not tied to a specific brand of patient handling equipment, since it is an autonomous device.
Can be added to any patient handling equipment without any influence on the medical device approval.
Makes the data readily accessible to a main control unit.
The main control unit, which stand in contact with the autonomous units, matches logged data with extended data, such as number of personnel and patients at a given time, to gain knowledge for decisions.
Typically, the system can be used for the following purposes, amongst others:
   Mapping of equipment usage within a ward over time for personnel education planning
   Mapping of equipment usage within a ward over time for Safe Patient Handling Act legislation following
   Mapping of equipment usage within a ward over time for (equipment) maintenance planning
   Mapping of equipment usage within a ward over time for equipment purchase planning
   Mapping of equipment usage for research and development feedback

An embodiment of the invention will now be described with reference to the following drawings, of which:
Figure 1 is a schematic of an accelerometer as used as a sensor in the present invention;
Figure 2 is a flow diagram of the system to collect and match the data relating to specific activities;
Figure 3 shows a trace of comparison of accelerometer readings during activities and no activity

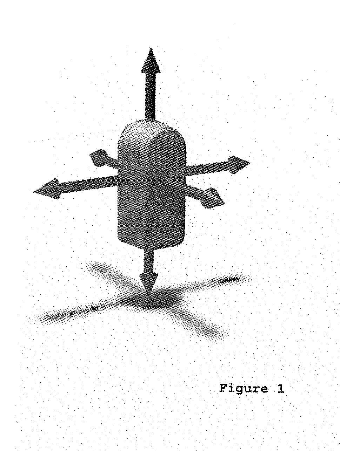

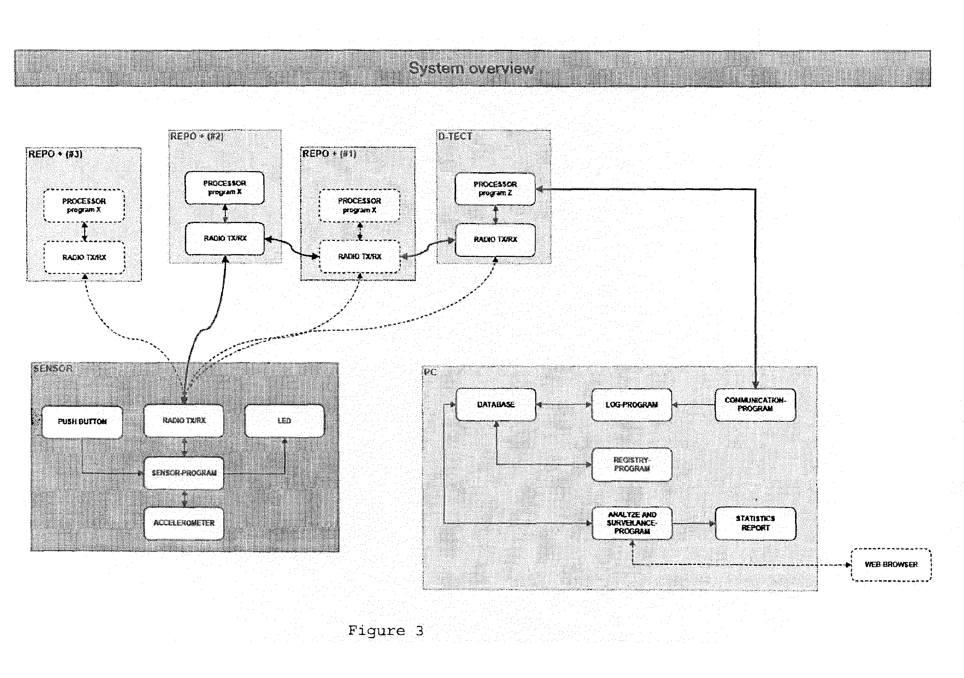

Referring to the Figures, an activity log unit including an a 3 axis accelerometer (Figure 1) is placed on the supervised equipment, such as patient handling equipment, the unit collecting measuring data (2) over time without compromising the function or integrity of the supervised equipment. The data collected is independent of mounting direction and placement on the equipment to be monitored. And it is not bound by any interface with any motors, hand controls or slings on the equipment. The accelerometer can be transported between wards with the equipment. As shown in Figure 3, an accelerometer together with a microprocessor and a transceiver forms a unit which are powered from a power source, this unit may have further functions such as buttons and/or displays.

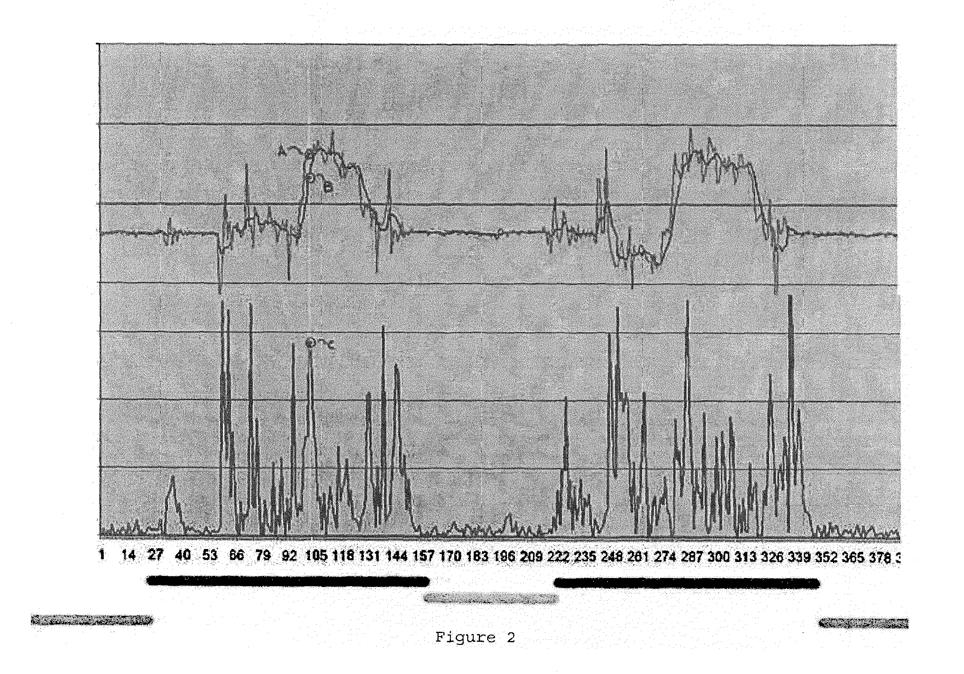

The accelerometer is polled in time intervals, the fetched accelerometer data, representing acceleration in g, is compared with time delayed data from the same accelerometer, when movement is introduced these two values (A and B in Figure 2) will differ much more than a non moving accelerometer (C in Figure 2). By comparing the two and introducing filters, a data vector representing the movement over time of the accelerometer is formed. The rules for interpreting all patient handling equipment are stored in the processor unit. This data vector can be compressed and sent to a main control unit over radio in several steps to reach the main control unit for further interpretation.

The microprocessor also runs a program that determines the different power modes of the unit, based upon accelerometer data, to conserve energy.

The main control unit time stamps all incoming data, making the need of a real time clock and calendar in each unit unnecessary. The main control unit stands in contact with the autonomous activity log units by radiocommunication and / or other suitable techniques.

The main control unit stores all relevant data from the data collecting units together with time and date and matches derived sensor data against experience data to sort out a specific event.

When the desired event is found, it is stored in a log (3) together with time and date. A main control unit then process this log-data together with the units ID-number and other relevant data to gain the advantage of such a system.

## Claims

1. An activity log system for a patient handling equipment, including an accelerometer together with a microprocessor and a transceiver powered from a power source mounted on the equipment, the accelerometer data relating to movement of the equipment collected and interpreted to identify specific equipment activities.

2. An activity log system as claimed in claim 1 **characterised in that** the specific equipment activities are interpreted by comparison with stored experimental data.

3. An activity log system as claimed in claims 1 or 2 **characterised in** the data collection is remote from the data interpretation.
